# EUROPÄISCHE PATENTSCHRIFT

(11) **EP 2 806 965 B1**
(45) Veröffentlichungstag und Bekanntmachung des Hinweises auf die Patenterteilung: **08.01.2020**
(21) Anmeldenummer: 12791485.1
(22) Anmeldetag: 28.11.2012
(51) Int. Cl.: B01D 53/14, C07C 227/18, C07C 229/12

(54) **VERFAHREN UND ABSORPTIONSMEDIUM ZUR ABSORPTION VON CO2 AUS EINER GASMISCHUNG**
METHOD AND ABSORPTION MEDIUM FOR ABSORBING CO2 FROM A GAS MIXTURE
PROCÉDÉ ET MILIEU D'ABSORPTION DESTINÉS À ABSORBER DU CO2 CONTENU DANS UN MÉLANGE GAZEUX

(30) Priorität: 23.01.2012 DE 102012200907
(43) Veröffentlichungstag der Anmeldung: 03.12.2014
(73) Patentinhaber: Evonik Operations GmbH, 45128 Essen (DE)
(72) Erfinder: SCHRAVEN, Alexander, 47661 Issum (DE); KNAUP, Günter, 63486 Bruchköbel (DE); SCHNEIDER, Rolf, 63584 Gründau-Rothenbergen (DE)
(74) Vertreter: Evonik Patent Association
(86) Internationale Anmeldenummer: PCT/EP2012/073778
(87) Internationale Veröffentlichungsnummer: WO 2013/110374

(56) Entgegenhaltungen:
- EP-A2- 0 079 767
- DE-A1- 2 123 773
- US-A- 4 405 586

## Beschreibung

Die Erfindung betrifft ein Verfahren und ein Absorptionsmedium zur Absorption von CO₂ aus einer Gasmischung.

In zahlreichen industriellen und chemischen Prozessen treten Gasströme auf, die einen unerwünschten Gehalt von CO₂ aufweisen, dessen Gehalt für die weitere Verarbeitung, für den Transport oder für eine Vermeidung von CO₂-Emissionen verringert werden muss.

Im industriellen Maßstab werden zur Absorption von CO₂ aus einer Gasmischung üblicherweise wässrige Kaliumcarbonatlösungen oder wässrige Lösungen von Alkanolaminen als Absorptionsmedium eingesetzt. Das beladene Absorptionsmedium wird durch Erwärmen, Entspannen auf einen niedrigeren Druck oder Strippen regeneriert, wobei das Kohlendioxid desorbiert wird. Nach dem Regenerationsprozess kann das Absorptionsmedium wieder verwendet werden. Diese Verfahren sind zum Beispiel in Kohl, A. L.; Nielsen, R. B., "Gas Purification", 5. Aufl., Gulf Publishing, Houston 1997 beschrieben.

Verfahren, die eine wässrige Lösung eines Alkanolamins als Absorptionsmedium verwenden, haben den Nachteil, dass zur Abtrennung von CO₂ durch Absorption und nachfolgende Desorption relativ viel Energie benötigt wird und dass Alkanolamine flüchtig sind, was eine Abtrennung von Alkanolamin aus dem Gas nach der Absorption erforderlich machen kann, um den Verlust an Amin zu reduzieren und eine Emission an umweltgefährdenden Stoffen zu vermeiden.

Verfahren, die eine wässrige Kaliumcarbonatlösung als Absorptionsmedium verwenden, haben den Nachteil, dass die Absorption von CO₂ in das Absorptionsmedium langsam erfolgt und dass in einem Zyklus aus Absorption und Desorption die als CO₂-Hub bezeichnete Kapazität des Absorptionsmediums gering ist.

Wässrige Lösungen von Kalium-N-methylalaninat und Kalium-N,N-dimethylglycinat wurden ab etwa 1935 unter den Handelsnamen Alkazid M und Alkazid DIK zur Absorption von CO₂ und H₂S aus Gasströmen eingesetzt, sind heute jedoch kaum noch in technischem Gebrauch. Wässrige Lösungen von Kalium-N,N-dimethylglycinat zeigen bei der Absorption von CO₂ eine zu langsame Aufnahme von CO₂ in das Absorptionsmedium. Kalium-N-methylalaninat ist bei der Absorption von CO₂ von den einfacher herzustellenden Alkanolaminen verdrängt worden.

In der Offenlegungsschrift DE 2123773 wird vorgeschlagen, zur Verbesserung des Stoffübergangs von CO₂ in die wässrige Kaliumcarbonatlösung als Aktivator eine N-Alkylaminosäure in einer Menge zwischen 2 und 5 Gew.-% zuzusetzen. Als dazu geeignete N-Alkylaminosäure wird unter anderen N-Isopropylglycin genannt. US 4,405,586 beschreibt zum gleichen Zweck N-sec.-Butylglycin als geeigneten Aktivator. US 4, 094, 957 beschreibt alternativ eine Mischung aus mindestens einem sterisch gehinderten Amin und einer Aminosäure als Additiv und US 4,405,579 beschreibt alternativ eine Mischung aus einer sterisch gehinderten monosubstituierten alpha-Aminosäure und einer tertiären alpha-Aminosäure als Additiv.

Der Zusatz von Aminosäuresalzen als Aktivator kann bei Verwendung einer wässrigen Kaliumcarbonatlösung zwar die Absorptionsrate verbessern, doch bleibt der Nachteil eines geringen CO₂-Hubs bestehen, aus dem sich hohe Massenströme an Absorptionsmedium und daraus folgend ein hoher Energieverbrauch zum Erhitzen des Absorptionsmediums bei der Desorption von CO₂ ergeben.

EP 0 079 767 A2 beschreibt die Herstellung von N-Isopropylglycin durch reduktive Aminierung von Aceton mit Glycin und Wasserstoff in wässriger Lösung in Gegenwart von Palladium auf Kohle als Hydrierkatalysator.

EP 0 187 130 beschreibt die Herstellung von N-Isopropylglycin durch reduktive Aminierung von Glyoxylsäure mit Isopropylamin und Wasserstoff in wässriger Lösung in Gegenwart von Palladium auf Kohle als Hydrierkatalysator.

JP 2002-047258 beschreibt die Herstellung von N-Isopropylglycin durch Verseifen von N-Isopropylglycinnitril mit Natronlauge zu Natrium-N-isopropylglycinat und anschließende Neutralisierung mit einem sauren Ionenaustauscher.

Es wurde nun gefunden, dass sich mit einem Absorptionsmedium, das Wasser und 5 bis 50 Gew.-% Aminosäuresalze der Formel R¹R²CHNHCH₂COOK enthält, wobei R¹ und R² n-Alkylreste mit zusammen 2 bis 4 Kohlenstoffatome sind, bei der Absorption von CO₂ aus einer Gasmischung gegenüber den bekannten Absorptionsmedien Alkazid M und Alkazid DIK eine technisch besser nutzbare Kombination aus hoher gewichtsbezogener CO₂-Absorptionskapazität im cyclischen Betrieb, rascher Absorption von CO₂ in das Absorptionsmedium sowie einfacher und abfallfreier Herstellbarkeit des Absorptionsmediums erreichen lässt.

Gegenstand der Erfindung ist deshalb ein Verfahren zur Absorption von CO₂ aus einer Gasmischung durch in Kontakt bringen der Gasmischung mit einem Absorptionsmedium, das mindestens 40 Gew.-% Wasser und 5 bis 50 Gew.-% Aminosäuresalze der Formel (I) (I) R¹R²CHNHCH₂COOK enthält, worin R¹ und R² n-Alkylreste sind und die Reste R¹ und R² zusammen 2 bis 4 Kohlenstoffatome aufweisen, und wobei das Absorptionsmedium zusätzlich Kaliumionen in Form von Kaliumcarbonat oder Kaliumhydrogencarbonat enthält und das molare Verhältnis von Kaliumionen in Form von Kaliumcarbonat oder Kaliumhydrogencarbonat zu Aminosäuresalzen der Formel (I) im Bereich von 0,01 bis 0,5 liegt.

Weitere Gegenstände der Erfindung sind das im erfindungsgemäßen Verfahren verwendete Absorptionsmedium sowie ein Verfahren zur Herstellung des erfindungsgemäßen Absorptionsmediums mit den Schritten
a) reduktive Aminierung mindestens eines Ketons der Formel (II)

   (II) R¹R²C=O

   worin R¹ und R² n-Alkylreste sind und die Reste R¹ und R² zusammen 2 bis 4 Kohlenstoffatome aufweisen, mit Glycin und Wasserstoff in wässriger Lösung in Gegenwart eines festen Hydrierkatalysators unter Bildung eines N-Alkylglycins,
b) Abtrennung des Hydrierkatalysators aus der in Schritt a) erhaltenen Mischung und
c) Zugabe von Kaliumhydroxid, Kaliumcarbonat oder Kaliumhydrogencarbonat zu der in Schritt b) erhaltenen Mischung, bis das molare Verhältnis von Kaliumionen in Form von Kaliumcarbonat oder Kaliumhydrogencarbonat zu Aminosäuresalzen der Formel (I) im Bereich von 0,01 bis 0,5 liegt.

Im erfindungsgemäßen Verfahren zur Absorption von CO₂ enthält das Absorptionsmedium 5 bis 50 Gew.-% Aminosäuresalze der Formel (I), vorzugsweise 10 bis 48 Gew.-% und besonders bevorzugt 15 bis 45 Gew.-% Aminosäuresalze der Formel (I). Weiter bevorzugt enthält das Absorptionsmedium 20 bis 45 Gew.-% und insbesondere 35 bis 45 Gew.-% Aminosäuresalze der Formel (I). Durch die Verwendung von Aminosäuresalzen der Formel (I) in diesen Mengenbereichen lässt sich ein hoher gewichtsbezogener CO₂-Hub, d.h. eine hohe gewichtsbezogene CO₂-Absorptionskapazität im cyclischen Betrieb von Absorption und Desorption erreichen. Gleichzeitig wird auch eine rasche Absorption von CO₂ in das Absorptionsmedium erzielt und die Gasmischung enthält nach der Absorption von CO₂ in das Absorptionsmedium keine umweltgefährdenden Bestandteile des Absorptionsmediums.

Als Aminosäuresalze der Formel (I) eignen sich Kalium-N-isopropylglycinat (R¹, R² = Methyl), Kalium-N-(sec-butyl)glycinat (R¹ = Ethyl, R² = Methyl), Kalium-N-(2-pentyl)glycinat (R¹ = n-Propyl, R² = Methyl) und Kalium-N-(3-pentyl)glycinat (R¹, R² = Ethyl) und Mischungen dieser Verbindungen. Vorzugsweise bestehen im Absorptionsmedium die Aminosäuresalze der Formel (I) zu mehr als 90 Gew.-% aus Kalium-N-isopropylglycinat. Die hohen Löslichkeiten von N-Isopropylglycin und Kalium-N-isopropylglycinat ermöglichen den Betrieb des erfindungsgemäßen Verfahrens mit einem hohen CO₂-Hub ohne Ausfällung von Aminosäure oder Aminosäuresalz.

Im erfindungsgemäßen Verfahren zur Absorption von CO₂ enthält das Absorptionsmedium zusätzlich zu Aminosäuresalzen der Formel (I) noch Kaliumcarbonat und/oder Kaliumhydrogencarbonat. Der Gehalt an Kaliumcarbonat und/oder Kaliumhydrogencarbonat wird so gewählt, dass das molare Verhältnis von Kaliumionen in Form von Kaliumcarbonat oder Kaliumhydrogencarbonat zu Aminosäuresalzen der Formel (I) im Bereich von 0,01 bis 0,5 liegt. Besonders bevorzugt liegt das molare Verhältnis im Bereich von 0,01 bis 0,1. Durch einen Gehalt an Kaliumcarbonat und/oder Kaliumhydrogencarbonat zusätzlich zu Aminosäuresalzen der Formel (I) lässt sich sicherstellen, dass sich eine Verunreinigung der Gasmischung durch Stickoxide und/oder Schwefeldioxid nicht nachteilig auf den CO₂-Hub des Absorptionsmediums auswirken, da diese Verunreinigungen nach Absorption in das Absorptionsmedium mit Kaliumcarbonat und/oder Kaliumhydrogencarbonat zu Kaliumnitrat beziehungsweise Kaliumsulfat umgesetzt werden und nicht zu einer Abnahme des Gehalts an Aminosäuresalzen der Formel (I) führen.

Im erfindungsgemäßen Verfahren zur Absorption von CO₂ enthält das Absorptionsmedium mindestens 40 Gew.-% Wasser. Das Absorptionsmedium kann zusätzlich zu Wasser und Aminosäuresalzen der Formel (I) noch ein oder mehrere physikalische Lösungsmittel enthalten. Der Anteil an physikalischen Lösungsmitteln kann dabei bis zu 20 Gew.-% betragen. Als physikalische Lösungsmittel eignen sich Sulfolan, aliphatische Säureamide, wie N-Formylmorpholin, N-Acetylmorpholin, N-Alkylpyrrolidone, insbesondere N-Methyl-2-pyrrolidon, oder N-Alkylpiperidone, sowie Diethylenglykol, Triethylenglykol und Polyethylenglykole und deren Alkylether, insbesondere Diethylenglykolmonobutylether. Vorzugsweise enthält das Absorptionsmedium jedoch kein physikalisches Lösungsmittel.

Das Absorptionsmedium kann zusätzlich noch Additive, wie Korrosionsinhibitoren, benetzungsfördernde Additive und Entschäumer aufweisen.

Als Korrosionsinhibitoren können im Absorptionsmedium alle Stoffe verwendet werden, die dem Fachmann zur Absorption von CO₂ unter Verwendung von Alkanolaminen als geeignete Korrosionsinhibitoren bekannt sind, insbesondere die in US 4,714,597 beschriebenen Korrosionsinhibitoren.

Als benetzungsförderndes Additiv werden vorzugsweise die aus WO 2010/089257 Seite 11, Zeile 18 bis Seite 13, Zeile 7 bekannten nichtionischen Tenside, zwitterionischen Tenside und kationischen Tenside verwendet.

Als Entschäumer können im Absorptionsmedium alle Stoffe verwendet werden, die dem Fachmann zur Absorption von CO₂ unter Verwendung von Alkanolaminen als geeignete Entschäumer bekannt sind.

Im erfindungsgemäßen Verfahren zur Absorption von CO₂ kann die Gasmischung ein Erdgas, ein Methan enthaltendes Biogas aus einer Fermentation, Kompostierung oder Kläranlage, ein Verbrennungsabgas, ein Abgas aus einer Kalzinierungsreaktion, wie dem Brennen von Kalk oder der Herstellung von Zement, ein Restgas aus einem Hochofenprozess zur Eisenherstellung oder eine aus einer chemischen Umsetzung resultierende Gasmischung, wie beispielsweise ein Kohlenmonoxid und Wasserstoff enthaltendes Synthesegas oder ein Reaktionsgas einer Wasserstoffherstellung durch Dampfreformieren sein. Vorzugsweise ist die Gasmischung ein Verbrennungsabgas, ein Erdgas oder ein Biogas, besonders bevorzugt ein Verbrennungsabgas, zum Beispiel aus einem Kraftwerk.

Die Gasmischung kann zusätzlich zu CO₂ noch weitere saure Gase enthalten, wie beispielsweise Stickoxide, COS, H₂S, CH₃SH oder SO₂. In einer bevorzugten Ausführungsform enthält die Gasmischung zusätzlich zu CO₂ noch H₂S. In einer weiteren bevorzugten Ausführungsform enthält die Gasmischung zusätzlich zu CO₂ noch Stickoxide und/oder SO₂. Ein Verbrennungsabgas wird vorzugsweise vorher entschwefelt, d.h. der Gehalt an SO₂ in der Gasmischung wird mit einem aus dem Stand der Technik bekannten Entschwefelungsverfahren, vorzugsweise durch eine Gaswäsche mit Kalkmilch, verringert, bevor das erfindungsgemäße Absorptionsverfahren durchgeführt wird.

Die Gasmischung weist vor dem in Kontakt bringen mit dem Absorptionsmedium vorzugsweise einen Gehalt an CO₂ im Bereich von 0,1 bis 50 Vol-% auf, besonders bevorzugt im Bereich von 1 bis 20 Vol-% und am meisten bevorzugt im Bereich von 8 bis 20 Vol-%.

Die Gasmischung kann zusätzlich zu CO₂ noch Sauerstoff enthalten, vorzugsweise mit einem Anteil von 0,1 bis 25 Vol-% und besonders bevorzugt mit einem Anteil von 0,1 bis 10 Vol-%.

Für das erfindungsgemäße Verfahren zur Absorption von CO₂ können alle zum in Kontakt bringen einer Gasphase mit einer Flüssigphase geeigneten Apparate verwendet werden, um die Gasmischung mit dem Absorptionsmedium in Kontakt zu bringen. Vorzugsweise werden aus dem Stand der Technik bekannte Gaswäscher oder Absorptionskolonnen verwendet, beispielsweise Membrankontaktoren, Radialstromwäscher, Strahlwäscher, Venturi-Wäscher, Rotations-Sprühwäscher Füllkörperkolonnen, Packungskolonnen oder Bodenkolonnen. Besonders bevorzugt werden Absorptionskolonnen im Gegenstrombetrieb verwendet.

Im erfindungsgemäßen Verfahren zur Absorption von CO₂ wird die Absorption vorzugsweise bei einer Temperatur des Absorptionsmediums im Bereich von 0 bis 80°C, besonders bevorzugt 20 bis 70°C, durchgeführt. Bei Verwendung einer Absorptionskolonne im Gegenstrombetrieb beträgt die Temperatur des Absorptionsmediums besonders bevorzugt 30 bis 60°C beim Eintritt in die Kolonne und 35 bis 70°C beim Austritt aus der Kolonne.

Vorzugsweise wird die CO₂ enthaltende Gasmischung bei einem anfänglichen Partialdruck von CO₂ von 0,01 bis 4 bar mit dem Absorptionsmedium in Kontakt gebracht. Besonders bevorzugt beträgt der anfängliche Partialdruck von CO₂ in der Gasmischung von 0,05 bis 3 bar. Der Gesamtdruck der Gasmischung liegt vorzugsweise im Bereich von 0,8 bis 50 bar, besonders bevorzugt 0,9 bis 30 bar.

In einer bevorzugten Ausführungsform des erfindungsgemäßen Verfahrens zur Absorption von CO₂ wird im Absorptionsmedium absorbiertes CO₂ durch Erhöhen der Temperatur und/oder Verringern des Drucks wieder desorbiert und das Absorptionsmedium nach dieser Desorption von CO₂ wieder zur Absorption von CO₂ verwendet. Vorzugsweise erfolgt die Desorption durch Erhöhen der Temperatur. Durch einen solchen zyklischen Prozess aus Absorption und Desorption kann CO₂ aus der Gasmischung ganz oder teilweise abgetrennt und getrennt von anderen Komponenten der Gasmischung erhalten werden.

Alternativ zum Erhöhen der Temperatur oder dem Verringern des Drucks oder zusätzlich zu einer Temperaturerhöhung und/oder Druckverringerung kann auch eine Desorption durch Strippen des mit CO₂ beladenen Absorptionsmediums mit einem inerten Gas, wie zum Beispiel Luft oder Stickstoff, durchgeführt werden.

Wenn bei der Desorption von CO₂ zusätzlich auch Wasser aus dem Absorptionsmedium entfernt wird, kann dem Absorptionsmedium vor der Wiederverwendung zur Absorption gegebenenfalls noch Wasser zugesetzt werden.

Für die Desorption können alle Apparate verwendet werden, die aus dem Stand der Technik zur Desorption eines Gases aus einer Flüssigkeit bekannt sind. Vorzugsweise wird die Desorption in einer Desorptionskolonne durchgeführt. Alternativ kann die Desorption von CO₂ auch in einer oder mehreren Flash-Verdampfungsstufen durchgeführt werden.

Die Desorption wird vorzugsweise bei einer Temperatur im Bereich von 50 bis 200°C durchgeführt. Bei einer Desorption durch Erhöhen der Temperatur wird die Desorption von CO₂ vorzugsweise bei einer Temperatur des Absorptionsmediums im Bereich von 50 bis 180°C, besonders bevorzugt 80 bis 150°C, durchgeführt. Die Temperatur bei der Desorption liegt dabei vorzugsweise mindestens 20°C, besonders bevorzugt mindestens 30°C, oberhalb der Temperatur bei der Absorption. Bevorzugt wird bei einer Desorption durch Erhöhen der Temperatur ein Strippen mit Wasserdampf, der durch Verdampfen eines Teils des Absorptionsmediums erzeugt wird, durchgeführt.

Bei einer Desorption durch Verringern des Drucks wird die Desorption vorzugsweise bei einem Druck im Bereich von 0,01 bis 10 bar durchgeführt.

In einer bevorzugten Ausführungsform des erfindungsgemäßen Verfahrens zur Absorption von CO₂ erfolgt die Desorption durch Strippen mit einem Inertgas, wie beispielsweise Luft oder Stickstoff, in einer Desorptionskolonne. Das Strippen in der Desorptionskolonne erfolgt vorzugsweise bei einer Temperatur des Absorptionsmediums im Bereich von 60 bis 100°C. Durch das Strippen kann mit geringem Energiebedarf ein niedriger Restgehalt des Absorptionsmediums an CO₂ nach Desorption erreicht werden.

In einer besonders bevorzugten Ausführungsform des erfindungsgemäßen Verfahrens zur Absorption von CO₂ werden die Schritte von Absorption und Desorption mehrfach wiederholt werden. Das Absorptionsmedium enthält zusätzlich Kaliumcarbonat und/oder Kaliumhydrogencarbonat und das molare Verhältnis von Kaliumionen in Form von Kaliumcarbonat oder Kaliumhydrogencarbonat zu Aminosäuresalzen der Formel (I) wird durch Zugabe von Kaliumhydroxid, Kaliumcarbonat oder Kaliumhydrogencarbonat im Bereich von 0,01 bis 0,5 gehalten. Vorzugsweise wird das molare Verhältnis durch Zugabe von Kaliumhydroxid im Bereich von 0,01 bis 0,5 gehalten, besonders bevorzugt im Bereich von 0,01 bis 0,1. Durch eine solche Zugabe von Kaliumhydroxid, Kaliumcarbonat oder Kaliumhydrogencarbonat lässt sich bei einer Absorption von CO₂ aus Gasmischungen, die Stickoxide und/oder Schwefeldioxid als Verunreinigung enthalten, auch über einen langen Zeitraum des cyclischen Betriebs eine Abnahme des CO₂-Hubs des Absorptionsmediums vermeiden.

Das erfindungsgemäße Absorptionsmedium zur Absorption von CO₂ aus einer Gasmischung umfasst mindestens 40 Gew.-% Wasser und 5 bis 50 Gew.-% Aminosäuresalze der Formel

(I) R¹R²CHNHCH₂COOK

worin R¹ und R² n-Alkylreste sind und die Reste R¹ und R² zusammen 2 bis 4 Kohlenstoffatome aufweisen, und umfasst zusätzlich Kaliumionen in Form von Kaliumcarbonat oder Kaliumhydrogencarbonat, wobei das molare Verhältnis von Kaliumionen in Form von Kaliumcarbonat oder Kaliumhydrogencarbonat zu Aminosäuresalzen der Formel (I) im Bereich von 0,01 bis 0,5 liegt. Vorzugsweise hat das erfindungsgemäße Absorptionsmedium eine Zusammensetzung, wie Sie voranstehend für das Absorptionsmedium von bevorzugten Ausführungsformen des erfindungsgemäßen Verfahrens zur Absorption von CO₂ beschrieben sind.

Das erfindungsgemäße Verfahren zur Herstellung eines Absorptionsmediums umfasst einen ersten Schritt a) einer reduktiven Aminierung mindestens eines Ketons der Formel R¹R²C=O, worin R¹ und R² n-Alkylreste sind und die Reste R¹ und R² zusammen 2 bis 4 Kohlenstoffatome aufweisen, mit Glycin und Wasserstoff. Geeignete Ketone der Formel R¹R²C=O sind Aceton, 2-Butanon, 2-Pentanon und 3-Pentanon und Mischungen dieser Verbindungen. Vorzugsweise wird als Keton der Formel R¹R²C=O Aceton eingesetzt. Die reduktive Aminierung erfolgt in wässriger Lösung in Gegenwart eines festen Hydrierkatalysators. Als Produkt der reduktiven Aminierung wird mindestens ein N-Alkylglycin der Formel R¹R²CHNHCH₂COOH erhalten. Mit Aceton als Keton der Formel R¹R²C=O wird als Produkt der reduktiven Aminierung N-Isopropylglycin erhalten.

Die reduktive Aminierung erfolgt vorzugsweise in einer wässrigen Lösung, die kein weiteres organisches Lösungsmittel zusätzlich zu Glycin und Ketonen der Formel R¹R²C=O enthält.

Als fester Hydrierkatalysator können alle dem Fachmann als zur reduktiven Aminierung von Ketonen geeignet bekannten heterogenen Katalysatoren eingesetzt werden. Vorzugsweise wird ein fester Hydrierkatalysator verwendet, der Palladium auf einem Trägermaterial, besonders bevorzugt Palladium auf Aktivkohleträger umfasst.

Die reduktive Aminierung wird vorzugsweise bei einer Temperatur von 0 bis 150 °C, besonders bevorzugt 40 bis 100 °C, durchgeführt. Der Wasserstoffpartialdruck wird bei der reduktiven Aminierung vorzugsweise in einem Bereich von 1 bis 30 bar, besonders bevorzugt 4 bis 15 bar, gehalten.

In einer bevorzugten Ausführungsform des erfindungsgemäßen Verfahrens zur Herstellung eines Absorptionsmediums wird im Schritt der reduktiven Aminierung der pH-Wert der wässrigen Lösung von Glycin und Ketonen der Formel R¹R²C=O vor Zugabe von Wasserstoff auf einen Wert im Bereich von 6 bis 10, vorzugsweise 8 bis 9, eingestellt.

Das erfindungsgemäße Verfahren zur Herstellung eines Absorptionsmediums umfasst im Anschluss an Schritt a) einen zweiten Schritt b), in dem der Hydrierkatalysator aus der in Schritt a) erhaltenen Mischung abgetrennt wird. Die Abtrennung des Hydrierkatalysators kann mit allem dem Fachmann bekannten Verfahren zur Fest-Flüssig-Trennung erfolgen, beispielsweise durch Filtrieren oder Zentrifugieren. Vorzugsweise wird der Hydrierkatalysator durch Filtrieren abgetrennt. Der abgetrennte Hydrierkatalysator kann in Schritt a) des Verfahrens wiederverwendet werden.

Die Schritte a) und b) des erfindungsgemäßen Verfahrens zur Herstellung eines Absorptionsmediums können auch in Form einer kontinuierlichen Umsetzung an einem Festbettkatalysator miteinander kombiniert werden, bei der eine wässrige Lösung von Glycin und Ketonen der Formel R¹R²C=O in Gegenwart von Wasserstoff über ein Festbett geleitet wird, das den Hydrierkatalysator in Form eines Festbettkatalysators enthält, wobei die Fest-Flüssig-Trennung von Schritt b) erfolgt, indem die flüssige Reaktionsmischung das Festbett verlässt.

Das erfindungsgemäße Verfahren zur Herstellung eines Absorptionsmediums umfasst im Anschluss an Schritt b) einen dritten Schritt c), in dem Kaliumhydroxid, Kaliumcarbonat oder Kaliumhydrogencarbonat zu der im zweiten Schritt erhaltenen katalysatorfreien Mischung gegeben wird. Vorzugsweise wird Kaliumhydroxid zugegeben. Kaliumhydroxid, Kaliumcarbonat oder Kaliumhydrogencarbonat werden vorzugsweise in einem molaren Überschuss zu dem in Schritt a) erhaltenen N-Alkylglycin zugegeben, um das N-Alkylglycin vollständig in das entsprechende Kalium-N-alkylglycinat umzusetzen. Es wird Kaliumhydroxid, Kaliumcarbonat oder Kaliumhydrogencarbonat zugegeben bis das molare Verhältnis von Kaliumionen in Form von Kaliumcarbonat oder Kaliumhydrogencarbonat zu Aminosäuresalzen der Formel (I) im Bereich von 0,01 bis 0,5 liegt.

Im Anschluss an Schritt b) oder Schritt c) kann optional Wasser aus der erhaltenen wässrigen Lösung entfernt werden, vorzugsweise durch Destillation, um in der in Schritt c) erhaltenen Lösung den für die Verwendung als Absorptionsmedium im erfindungsgemäßen Verfahren zur Absorption von CO₂ gewünschten Gehalt an Aminosäuresalzen der Formel (I) einzustellen. Zusammen mit Wasser können dabei auch nicht umgesetzte Ketone der Formel R¹R²C=O entfernt werden.

Mit dem erfindungsgemäßen Verfahren zur Herstellung des Absorptionsmediums lässt sich ein erfindungsgemäßes Absorptionsmedium in einfacher Weise aus leicht und in großen Mengen zugänglichen Ausgangsstoffen herstellen. Das Verfahren kann ohne Reinigungsschritte und praktisch ohne Bildung von Abfällen durchgeführt werden und erfordert nur wenige Apparate. Die in Schritt c) erhaltene Lösung kann ohne weitere Reinigung als Absorptionsmedium in dem erfindungsgemäßen Verfahren zur Absorption von CO₂ eingesetzt werden.

Die nachfolgenden Beispiele verdeutlichen die Erfindung, ohne jedoch den Gegenstand der Erfindung zu beschränken.

### Beispiele

### Beispiel 1

### Herstellung einer wässrigen Lösung von Kalium-N-isopropylglycinat

37,52 g Glycin wurden in einer Mischung aus 500 ml Wasser und 147 ml Aceton gelöst. Der pH-Wert der Lösung wurde dann durch Zugabe von 1,49 g 85 Gew.-% Kaliumhydroxid auf einen Wert von 8,5 eingestellt. Nach Zugabe von 10 g 5 Gew.-% Palladium auf Aktivkohle (50 Gew.-% wasserfeucht) wurde mit Wasserstoff auf 6 bar aufgedrückt und die Mischung bei konstantem Wasserstoffdruck von 6 bar 14 h bei 55°C gerührt. Anschließend wurde der Katalysator durch Vakuumfiltration abgetrennt. Ein ¹H-NMR-Spektrum der erhaltenen Lösung zeigte als Reaktionsprodukte von Glycin N-Isopropylglycin und N,N-Diisopropylglycin in einem molaren Verhältnis von 50:1. Die Lösung wurde am Rotationsverdampfer auf ca. 100 ml eingeengt. Danach wurden 31,6 g 85 Gew.-% Kaliumhydroxid zugegeben und die Mischung mit Wasser auf 222 g aufgefüllt.

### Beispiel 2

### Herstellung einer wässrigen Lösung von Kalium-N-(sec-butyl)glycinat

75,1 g Glycin wurden in 450 ml Wasser gelöst und der pH-Wert der Lösung wurde durch Zugabe von 3,3 g 85 Gew.-% Kaliumhydroxid auf einen Wert von 8,5 eingestellt. Nach Zugabe von 108,2 g 2-Butanon und 7,5 g 5 Gew.-% Palladium auf Aktivkohle (50 Gew.-% wasserfeucht) wurde mit Wasserstoff auf 5 bar aufgedrückt und die Mischung bei konstantem Wasserstoffdruck von 5 bar 48 h bei 55°C gerührt. Anschließend wurde der Katalysator durch Vakuumfiltration abgetrennt. Ein ¹H-NMR-Spektrum der erhaltenen Lösung zeigte als Reaktionsprodukt von Glycin N-sec-Butylylglycin und nicht umgesetztes Glycin in einem molaren Verhältnis von 50:1. Die Lösung wurde am Rotationsverdampfer auf ca. 200 ml eingeengt. Danach wurden 60 g 85 Gew.-% Kaliumhydroxid zugegeben und die Mischung mit Wasser auf 500 g aufgefüllt.

### Beispiele 3 bis 17

### Bestimmung der Absorptionskapazität für CO₂

Zur Bestimmung der CO₂-Beladung und des CO₂-Hubs wurden 150 g wässriges Absorptionsmedium mit den in Tabelle 1 angeführten Anteilen an Aminosäure und Kaliumhydroxid in einem thermostatisierbaren Behälter mit aufgesetztem und auf 3°C gekühltem Rückflusskühler vorgelegt. Nach Aufheizen auf 40°C bzw. 100°C wurde über eine Fritte am Behälterboden eine Gasmischung aus 14 Vol-% CO₂, 80 Vol-% Stickstoff und 6 Vol-% Sauerstoff mit einer Flussrate von 59 l/h durch das Absorptionsmedium geleitet und die CO₂-Konzentration im aus dem Rückflusskühler austretenden Gasstrom über die IR-Absorption mit einem CO₂-Analysator bestimmt. Durch Integration der Differenz des CO₂-Gehalts im eingeleiteten Gasstrom und im austretenden Gasstrom wurde die aufgenommene CO₂-Menge ermittelt und die Gleichgewichtsbeladung des Absorptionsmediums mit CO₂ berechnet. Der CO₂-Hub wurde als Differenz der bei 40°C und 100°C aufgenommenen CO₂-Mengen berechnet. Die so bestimmten Gleichgewichtsbeladungen bei 40 und 100°C in mol CO₂ / kg Absorptionsmedium und der CO₂-Hub in mol CO₂ / kg Absorptionsmedium sind in Tabelle 1 angeführt.

**Tabelle 1**

| Beispiel | Aminosäure | KOH | Beladung bei 40°C in mol/kg | Beladung bei 100°C in mol/kg | CO₂-Hub in mol/kg |
|---|---|---|---|---|---|
| 3* | 30,0 g Glycin | 26,5 g | 1,74 | 1,33 | 0,41 |
| 4* | 30,0 g N-Methylglycin | 22,5 g | 1,74 | 1,16 | 0,58 |
| 5* | 30,0 g Ethylglycin | 19,5 g | 1,92 | 1,08 | 0,84 |
| 6* | 30,0 g N,N-Dimethylglycin | 19,5 g | 1,78 | 0,67 | 1,11 |
| 7* | 30,0 g N-Propylglycin | 17,1 g | 1,54 | 0,93 | 0,61 |
| 8 | 30,0 g N-Isopropylglycin | 17,1 g | 1,81 | 0,82 | 0,99 |
| 9 | 30,0 g N-(sec-Butyl)glycin | 14,3 g | 1,44 | 0,63 | 0,81 |
| 10 | 30,0 g N-(3-Pentyl)glycin | 13,6 g | 1,32 | 0,54 | 0,78 |
| 11 | 37,6 g N-Isopropylglycin | 21,4 g | 2,16 | 0,84 | 1,32 |
| 12* | 45,0 g Glycin | 33,4 g | 2,91 | 2,23 | 0,68 |
| 13* | 45,0 g N-Methylglycin | 33,5 g | 2,43 | 1, 65 | 0,78 |
| 14* | 45,0 g N-Methylalanin | 30,0 g | 2,71 | 1,51 | 1,20 |
| 15* | ohne | 25,7 g | 2,57 | 2,18 | 0,39 |
| 16* | 7,5 g N-Methylalanin | 25,7 g | 2,55 | 1,87 | 0,68 |
| 17* | 45,0 g N-Methylalanin | 25,7 g | 2,47 | 1,33 | 1,14 |

| | | | | | |
|---|---|---|---|---|---|
| *nicht erfindungsgemäß | | | | | |

## Patentansprüche

1. Verfahren zur Absorption von CO₂ aus einer Gasmischung durch in Kontakt bringen der Gasmischung mit einem Absorptionsmedium, **dadurch gekennzeichnet, dass** das Absorptionsmedium mindestens 40 Gew.-% Wasser und 5 bis 50 Gew.-% Aminosäuresalze der Formel (I)
(I) R¹R²CHNHCH₂COOK
enthält, worin R¹ und R² n-Alkylreste sind und die Reste R¹ und R² zusammen 2 bis 4 Kohlenstoffatome aufweisen, und wobei das Absorptionsmedium zusätzlich Kaliumionen in Form von Kaliumcarbonat oder Kaliumhydrogencarbonat enthält und das molare Verhältnis von Kaliumionen in Form von Kaliumcarbonat oder Kaliumhydrogencarbonat zu Aminosäuresalzen der Formel (I) im Bereich von 0,01 bis 0,5 liegt.

2. Verfahren nach Anspruch 1, **dadurch gekennzeichnet, dass** das Absorptionsmedium 10 bis 48 Gew.-% und vorzugsweise 35 bis 45 Gew.-% Aminosäuresalze der Formel (I) enthält.

3. Verfahren nach Anspruch 1 oder 2, **dadurch gekennzeichnet, dass** die Aminosäuresalze der Formel (I) zu mehr als 90 Gew.-% Kalium-N-isopropylglycinat sind.

4. Verfahren nach einem der Ansprüche 1 bis 3, **dadurch gekennzeichnet, dass** die Gasmischung ein Verbrennungsabgas, ein Erdgas oder ein Biogas ist.

5. Verfahren nach einem der Ansprüche 1 bis 4, **dadurch gekennzeichnet, dass** im Absorptionsmedium absorbiertes CO₂ durch Erhöhen der Temperatur und/oder Verringern des Drucks wieder desorbiert wird und das Absorptionsmedium nach dieser Desorption von CO₂ wieder zur Absorption von CO₂ verwendet wird.

6. Verfahren nach Anspruch 5, **dadurch gekennzeichnet, dass** die Absorption bei einer Temperatur im Bereich von 0 bis 80°C und die Desorption bei einer höheren Temperatur im Bereich von 50 bis 200°C durchgeführt wird.

7. Verfahren nach Anspruch 5 oder 6, **dadurch gekennzeichnet, dass** die Absorption bei einem Druck im Bereich von 0,8 bis 50 bar und die Desorption bei einem niedrigeren Druck im Bereich von 0,01 bis 10 bar durchgeführt wird.

8. Verfahren nach einem der Ansprüche 5 bis 7, **dadurch gekennzeichnet, dass** die Schritte von Absorption und Desorption mehrfach wiederholt werden, und das molare Verhältnis von Kaliumionen in Form von Kaliumcarbonat oder Kaliumhydrogencarbonat zu Aminosäuresalzen der Formel (I) durch Zugabe von Kaliumhydroxid, Kaliumcarbonat oder Kaliumhydrogencarbonat im Bereich von 0,01 bis 0,5 gehalten wird.

9. Absorptionsmedium zur Absorption von CO₂ aus einer Gasmischung umfassend mehr als 40 Gew.-% Wasser und 5 bis 50 Gew.-% Aminosäuresalze der Formel
(I) R¹R²CHNHCH₂COOK
worin R¹ und R² n-Alkylreste sind und die Reste R¹ und R² zusammen 2 bis 4 Kohlenstoffatome aufweisen, wobei das Absorptionsmedium zusätzlich Kaliumionen in Form von Kaliumcarbonat oder Kaliumhydrogencarbonat umfasst und das molare Verhältnis von Kaliumionen in Form von Kaliumcarbonat oder Kaliumhydrogencarbonat zu Aminosäuresalzen der Formel (I) im Bereich von 0,01 bis 0,5 liegt.

10. Absorptionsmedium nach Anspruch 9, **dadurch gekennzeichnet, dass** es 10 bis 48 Gew.-% und vorzugsweise 35 bis 45 Gew.-% Aminosäuresalze der Formel (I) umfasst.

11. Absorptionsmedium nach Anspruch 9 oder 10, **dadurch gekennzeichnet, dass** die Aminosäuresalze der Formel (I) zu mehr als 90 Gew.-% Kalium-N-isopropylglycinat sind.

12. Verfahren zur Herstellung eines Absorptionsmediums gemäß einem der Ansprüche 9 bis 11, umfassend die Schritte
a) reduktive Aminierung mindestens eines Ketons der Formel (II)
(II) R¹R²C=O
worin R¹ und R² n-Alkylreste sind und die Reste R¹ und R² zusammen 2 bis 4 Kohlenstoffatome aufweisen, mit Glycin und Wasserstoff in wässriger Lösung in Gegenwart eines festen Hydrierkatalysators unter Bildung eines N-Alkylglycins,
b) Abtrennung des Hydrierkatalysators aus der in Schritt a) erhaltenen Mischung und
c) Zugabe von Kaliumhydroxid, Kaliumcarbonat oder Kaliumhydrogencarbonat zu der in Schritt b) erhaltenen katalysatorfreien Mischung, bis das molare Verhältnis von Kaliumionen in Form von Kaliumcarbonat oder Kaliumhydrogencarbonat zu Aminosäuresalzen der Formel (I) im Bereich von 0,01 bis 0,5 liegt.

13. Verfahren nach Anspruch 12, **dadurch gekennzeichnet, dass** das Keton der Formel (II) Aceton ist.

14. Verfahren nach einem der Ansprüche 12 bis 13, **dadurch gekennzeichnet, dass** in Schritt c) Kaliumhydroxid zugegeben wird.

15. Verfahren nach einem der Ansprüche 12 bis 14, **dadurch gekennzeichnet, dass** Schritt a) bei einer Temperatur von 0 bis 150 °C und einem Wasserstoffpartialdruck von 1 bis 30 bar durchgeführt wird.

16. Verfahren nach einem der Ansprüche 12 bis 15, **dadurch gekennzeichnet, dass** in Schritt a) der pH-Wert der wässrigen Lösung von Glycin und Ketonen der Formel (II) vor Zugabe von Wasserstoff auf einen Wert im Bereich von 6 bis 10, vorzugsweise 8 bis 9, eingestellt wird.

## Claims

1. Process for absorbing CO₂ from a gas mixture by contacting the gas mixture with an absorption medium, **characterized in that** the absorption medium comprises at least 40 wt% of water and 5 to 50 wt% of amino acid salts of formula (I)
(I) R¹R²CHNHCH₂COOK
in which R¹ and R² are n-alkyl radicals, and the radicals R¹ and R² together have 2 to 4 carbon atoms, and wherein the absorption medium further comprises potassium ions in the form of potassium carbonate or potassium hydrogen carbonate, and the molar ratio of potassium ions in the form of potassium carbonate or potassium hydrogen carbonate to amino acid salts of formula (I) is in the range from 0.01 to 0.5.

2. Process according to Claim 1, **characterized in that** the absorption medium comprises 10 to 48 wt% and preferably 35 to 45 wt% of amino acid salts of formula (I).

3. Process according to Claim 1 or 2, **characterized in that** more than 90 wt% of the amino acid salts of formula (I) are potassium N-isopropylglycinate.

4. Process according to any one of Claims 1 to 3, **characterized in that** the gas mixture is a combustion off-gas, a natural gas or a biogas.

5. Process according to any one of Claims 1 to 4, **characterized in that** CO₂ absorbed in the absorption medium is desorbed again by increasing the temperature and/or reducing the pressure and the absorption medium after this desorption of CO₂ is used again for absorbing CO₂.

6. Process according to Claim 5, **characterized in that** the absorption is carried out at a temperature in the range from 0 to 80°C and the desorption is carried out at a higher temperature in the range from 50 to 200°C.

7. Process according to Claim 5 or 6, **characterized in that** the absorption is carried out at a pressure in the range from 0.8 to 50 bar and the desorption is carried out at a lower pressure in the range from 0.01 to 10 bar.

8. Process according to any one of Claims 5 to 7, **characterized in that** the steps of absorption and desorption are repeated several times, and the molar ratio of potassium ions in the form of potassium carbonate or potassium hydrogen carbonate to amino acid salts of formula (I) is maintained in the range from 0.01 to 0.5 by addition of potassium hydroxide, potassium carbonate or potassium hydrogen carbonate.

9. Absorption medium for absorbing CO₂ from a gas mixture, comprising more than 40 wt% of water and 5 to 50 wt% of amino acid salts of formula
(I) R¹R²CHNHCH₂COOK
in which R¹ and R² are n-alkyl radicals, and the radicals R¹ and R² together have 2 to 4 carbon atoms, wherein the absorption medium further comprises potassium ions in the form of potassium carbonate or potassium hydrogen carbonate, and the molar ratio of potassium ions in the form of potassium carbonate or potassium hydrogen carbonate to amino acid salts of formula (I) is in the range from 0.01 to 0.5.

10. Absorption medium according to Claim 9, **characterized in that** it comprises 10 to 48 wt% and preferably 35 to 45 wt% of amino acid salts of formula (I).

11. Absorption medium according to Claim 9 or 10, **characterized in that** more than 90 wt% of the amino acid salts of formula (I) are potassium N-isopropylglycinate.

12. Process for preparing an absorption medium according to any one of Claims 9 to 11, comprising the steps of
a) reductively aminating at least one ketone of formula (II)
(II) R¹R²C=O
in which R¹ and R² are n-alkyl radicals, and the radicals R¹ and R² together have 2 to 4 carbon atoms, with glycine and hydrogen in aqueous solution, in the presence of a solid hydrogenation catalyst, to form an n-alkyl glycine,
b) removing the hydrogenation catalyst from the mixture obtained in step a), and
c) adding potassium hydroxide, potassium carbonate or potassium hydrogen carbonate to the catalyst-free mixture obtained in step b) until the molar ratio of potassium ions in the form of potassium carbonate or potassium hydrogen carbonate to amino acid salts of formula (I) is in the range from 0.01 to 0.5.

13. Process according to Claim 12, **characterized in that** the ketone of formula (II) is acetone.

14. Process according to either of Claims 12 and 13, **characterized in that** potassium hydroxide is added in step c).

15. Process according to any one of Claims 12 to 14, **characterized in that** step a) is carried out at a temperature of 0 to 150°C and at a hydrogen partial pressure of 1 to 30 bar.

16. Process according to any one of Claims 12 to 15, **characterized in that** in step a) the pH of the aqueous solution of glycine and ketones of formula II is adjusted to a level in the range from 6 to 10, preferably 8 to 9, before hydrogen is added.

## Revendications

1. Procédé pour l'absorption de CO₂ d'un mélange de gaz par la mise en contact du mélange de gaz avec un milieu d'absorption, **caractérisé en ce que** le milieu d'absorption contient au moins 40% en poids d'eau et 5 à 50% en poids de sels d'acide aminé de formule (I)
(I) R¹R²CHNHCH₂COOK
dans laquelle R¹ et R² sont des radicaux n-alkyle et les radicaux R¹ et R² présentent ensemble 2 à 4 atomes de carbone, et le milieu d'absorption contenant de plus des ions potassium sous forme de carbonate de potassium ou d'hydrogénocarbonate de potassium et le rapport molaire des ions potassium sous forme de carbonate de potassium ou d'hydrogénocarbonate de potassium aux sels d'acide aminé de formule (I) se situant dans la plage de 0,01 à 0,5.

2. Procédé selon la revendication 1, **caractérisé en ce que** le milieu d'absorption contient 10 à 48% en poids et de préférence 35 à 45% en poids de sels d'acide aminé de formule (I).

3. Procédé selon la revendication 1 ou 2, **caractérisé en ce que** le N-isopropylglycinate de potassium représente plus de 90% en poids des sels d'acide aminé de formule (I).

4. Procédé selon l'une quelconque des revendications 1 à 3, **caractérisé en ce que** le mélange de gaz est un gaz d'échappement de combustion, un gaz naturel ou un biogaz.

5. Procédé selon l'une quelconque des revendications 1 à 4, **caractérisé en ce que** le CO₂ absorbé dans le milieu d'absorption est à nouveau désorbé par élévation de la température et/ou par diminution de la pression et le milieu d'absorption, après cette désorption de CO₂, est réutilisé pour l'absorption de CO₂.

6. Procédé selon la revendication 5, **caractérisé en ce que** l'absorption est réalisée à une température dans la plage de 0 à 80°C et la désorption est réalisée à une température plus élevée dans la plage de 50 à 200°C.

7. Procédé selon la revendication 5 ou 6, **caractérisé en ce que** l'absorption est réalisée à une pression dans la plage de 0,8 à 50 bars et la désorption est réalisée à une pression plus basse dans la plage de 0,01 à 10 bars.

8. Procédé selon l'une quelconque des revendications 5 à 7, caractérisé en ce les étapes d'absorption et de désorption sont répétées plusieurs fois, et le rapport molaire des ions potassium sous forme de carbonate de potassium ou d'hydrogénocarbonate de potassium aux sels d'acide aminé de formule (I) est maintenu dans la plage de 0,01 à 0,5 par l'ajout d'hydroxyde de potassium, de carbonate de potassium ou d'hydrogénocarbonate de potassium.

9. Milieu d'absorption pour l'absorption de CO₂ d'un mélange de gaz comprenant plus de 40% en poids d'eau et 5 à 50% en poids de sels d'acide aminé de formule
(I) R¹R²CHNHCH₂COOK
dans laquelle R¹ et R² sont des radicaux n-alkyle et les radicaux R¹ et R² présentent ensemble 2 à 4 atomes de carbone, et le milieu d'absorption contenant de plus des ions potassium sous forme de carbonate de potassium ou d'hydrogénocarbonate de potassium et le rapport molaire des ions potassium sous forme de carbonate de potassium ou d'hydrogénocarbonate de potassium aux sels d'acide aminé de formule (I) se situant dans la plage de 0,01 à 0,5.

10. Milieu d'absorption selon la revendication 9, **caractérisé en ce qu'**il contient 10 à 48% en poids et de préférence 35 à 45% en poids de sels d'acide aminé de formule (I).

11. Milieu d'absorption selon la revendication 9 ou 10, **caractérisé en ce que** le N-isopropylglycinate de potassium représente plus de 90% en poids des sels d'acide aminé de formule (I).

12. Procédé pour la préparation d'un milieu d'absorption selon l'une quelconque des revendications 9 à 11, comprenant les étapes de
a) amination réductrice d'au moins une cétone de formule (II)
(II) R¹R²C=O
dans laquelle R¹ et R² sont des radicaux n-alkyle et les radicaux R¹ et R² présentent ensemble 2 à 4 atomes de carbone,
avec de la glycine et de l'hydrogène en solution aqueuse en présence d'un catalyseur solide d'hydrogénation avec formation d'une N-alkylglycine,
b) séparation du catalyseur d'hydrogénation du mélange obtenu dans l'étape a) et
c) ajout d'hydroxyde de potassium, de carbonate de potassium ou d'hydrogénocarbonate de potassium au mélange exempt de catalyseur obtenu dans l'étape b), jusqu'à ce que le rapport molaire des ions potassium sous forme de carbonate de potassium ou d'hydrogénocarbonate de potassium aux sels d'acide aminé de formule (I) se situe dans la plage de 0,01 à 0,5.

13. Procédé selon la revendication 12, **caractérisé en ce que** la cétone de formule (II) est l'acétone.

14. Procédé selon l'une quelconque des revendications 12 et 13, **caractérisé en ce que** de l'hydroxyde de potassium est ajouté dans l'étape c).

15. Procédé selon l'une quelconque des revendications 12 à 14, **caractérisé en ce que** l'étape a) est réalisée à une température de 0 à 150°C et à une pression partielle d'hydrogène de 1 à 30 bars.

16. Procédé selon l'une quelconque des revendications 12 à 15, **caractérisé en ce que** dans l'étape a), la valeur de pH de la solution aqueuse de glycine et de cétones de formule (II), avant l'ajout d'hydrogène, est ajustée à une valeur dans la plage de 6 à 10, de préférence de 8 à 9.
